# EUROPEAN PATENT APPLICATION

(11) **EP 4 537 742 A1**
(43) Date of publication of application: **16.04.2025**
(21) Application number: 23306778.4
(22) Date of filing: 12.10.2023
(51) Int. Cl.: A61B 5/00, A61B 17/34

(54) **DETECTION DEVICE FOR USE IN DETECTION OF A LEAKAGE IN AN ANATOMICAL CONDUIT**

(71) Applicant: Qaelon Medical, 67000 Strasbourg (FR)
(72) Inventor: BETTING, Fabienne, 67000 Strasbourg (FR); MARDON, Ariel, 67000 Strasbourg (FR)
(74) Representative: Cabinet Nuss

(57) **Abstract**

The present invention relates to a hand-held detection device (1) for use in detection of a leakage in an anatomical conduit within a cavity of a body part filled with a gas mixture, the detection device (1) comprising:
- an access element (10) configured to penetrate the body part, and a housing (20), both configured to be held by hand of a user,
- a detection module (30) comprising:
- a sensor configured to measure at least one gas mixture parameter related to the gas mixture within the cavity,
- a computational module arranged in the housing (20) and connected to said at least one sensor, and being configured to compute the gas mixture parameter to provide a gas information, a user interface (33) being configured to communicate said gas information.

## Description

### FIELD OF INVENTION

The present disclosure relates to a detection device for use in providing a gas information from a gas mixture contained within a cavity of a body part of a living being, as during the detection of a leakage in an anatomical conduit. The present disclosure also relates to a system for the detection of a leakage comprising said device, and a method of use of said device.

The disclosure specifically applies to the field of medical procedures, methods, systems and devices, more specifically minimally invasive surgical procedures and diagnostic systems to evaluate the integrity of an organ located in a body cavity, in particular of a tubular or hollow organ, as well as the size of a perforation or of an incomplete closure during the course of a surgical procedure.

### BACKGROUND OF INVENTION

It is known from prior art systems to monitor the integrity of an organ during a surgery, e.g., a laparoscopy.

Document US2015272499, in the name of the Applicant, describes a system for detecting leaks and/or verifying adequate closure, following a medical procedure on a hollow or tubular organ of a subject. The system comprises:
- an injection module for insufflating, in the concerned organ, a specific test gas which is not commonly or naturally present or produced within the body of the subject, or which is present or produced in a precisely known amount or concentration, and
- a detection module for analyzing percutaneously the gas mixture locally present within the body cavity in which said organ is situated.

In cooperation with a computational module, the presence or the concentration of the injected test gas in the gas mixture indicates whether or not the concerned organ or a lumen defined by the latter is leak free or not.

But this kind of system, although efficient, can be cumbersome to implement: all the modules of the system are driven by a single computational module, which is by consequence complex and remote.

Moreover, this kind of system being thorough, all the modules need to be interoperable and work together thus complexifying each module.

### SUMMARY

The aim of the invention is to overcome the drawbacks of the prior art by proposing simplified system wherein a least a module is independent from the other, nevertheless allowing a gas information surveillance as done in the prior art.

To this end, it has been developed a detection device for use in providing a gas information from a gas mixture contained within a cavity of a body part of a living being, and the body part comprising a skin layer delimiting the cavity, the detection device comprising:
- an access element presenting a distal end, and configured to be in communication with the cavity,
- a housing connected to the access element,
- a detection module.

According to the invention, the access element and the housing are configured to be held by hand of a user, and the detection module comprises:
- at least one sensor comprising a gas sensor arranged on the access element and configured to measure at least one gas mixture parameter related to the gas mixture within the cavity,
- a computational module arranged in the housing and connected to said at least one sensor, the computational module being configured to compute the gas mixture parameter to provide the gas information,
- a user interface arranged in the housing and connected to the computational module, the user interface configured to communicate gas information,
- a power supply arranged in the housing and configured to supply the detection module with energy.

In this way, the detection device is autonomous and can work independently, with its own sensor, computational module, power supply and user interface. It allows a simple and reliable gas information detection.

Moreover, it can cooperate with surgery systems of the prior art which are not equipped with a leakage detection module. There is no more need to complexify the computational modules of surgery systems, given they do not need to operate the leak detection device.

Preferably, the gas information is representative of a leakage in an anatomical conduit within the cavity.

In order to be in communication with the cavity, the access element can penetrate the body part through the skin, and reach by itself the cavity.

In a preferred embodiment, the detection device is configured for use with a trocar having a cannula defining a passage to a free end and intended to be inserted in the body part through the skin layer, wherein the access element is configured to penetrate the cannula with the gas sensor in fluid communication with the passage, so that the gas sensor is in contact with the gas mixture when the cannula is arranged within the cavity. In this way, the detection device can be easily put in place on the patient, through a preinstalled trocar, e.g., a preinstalled trocar for surgical instruments to access the body cavity during the surgery. There is no need to perform further surgical act on the body to install the detection device, and once the detection is made, the device can be put out and the surgery can carry on, the trocar being free for receiving back the surgical instruments.

In this case, the access element does not need to penetrate the body part and reach the cavity, because the cannula of the trocar does this. By being inserted in the cannula, the access element is in communication with the cavity.

In order to guarantee the holding of the device on the trocar, and prevent any unintentional disassembly or fall of the detection device from the trocar, an adjustment member is disposed on an outer surface of the access element, and is configured to be radially tight against an internal surface of the cannula.

Preferably, the access element is configured to be radially tight against a sealing valve of the trocar, in order to prevent gas leakage from the body cavity, through a gap between the access element and the valve.

In the preferred embodiment, a switch controlling the activation and the deactivation of the detection module is arranged on an external surface of the detection module, and is configured to be actuated by the trocar when the detection module is mounted on the trocar. It allows to automatically power on the detection device when plugging it into the trocar, then power off the detection device when taking it out. A such feature allows a "plug and play" detection device, which is very convenient and quick to use.

Advantageously, a sealing member on an outer surface of the access element is configured to obturate a connection hole of the trocar, when the access element penetrates the cannula. This allows, when the installed trocar is of the type with a connection hole, to prevent gas leakage from the body cavity through said hole during the installation or the use of the detection device.

In an embodiment, the computational module is configured to communicate and receive an external parameter from an external sensor, and the computational module is configured to compute the external parameter along with the gas mixture parameter. This allows to run a more detailed leakage analysis, by including a further parameter.

For a similar purpose, the device can comprise a supplementary sensor configured to measure at least one supplementary parameter, and the computational module is configured to compute the supplementary parameter along with the gas mixture parameter.

In order to generate and to guide a flow of the gas mixture, from the body cavity to the exterior of the body, the access element comprises a gas outlet at a proximal end opposite the distal end.

In order to compute more parameters during the detection, without complexifying the computational module of the insufflator of the system, the detection device comprises a gas connection device configured to:
- communicate with a test gas insufflator, and
- receive a test gas parameter,
and the computational module is configured to compute the test gas parameter along with the gas mixture parameter.

In order to ease the handling of the detection device, the access element presents a proximal end opposite the distal end, the housing being attached to the proximal end of the access element.

Preferably, the housing comprises a data storage, configured to store gas parameter data from the sensor, or configured to store gas information. This allows to collect and use data a posteriori, notably when the surgery is conducted in a room shielded against radiations, as X-rays, which also prevent any wireless communication between the detection device and a ranged computer placed outside the room.

The invention also concerns a system comprising:
- a trocar, comprising a cannula which is inserted, through a skin layer delimiting a cavity of a body part of a living being, so that the cannula is arranged within the cavity which is filled by a gas mixture; and
- a handheld detection device according to above features, and configured to be inserted in the cannula so that the distal end is in communication with the cavity, and so that the sensor measures at least one gas mixture parameter related to the gas mixture.

A such system may comprise an insufflator intended to inject a test gas into an anatomical conduit within the cavity so that the sensor measures at least one gas mixture parameter related to the test gas.

The invention also concerns a method of use of a detection device for detecting a leakage in an anatomical conduit within a cavity of a body part of a living being, the cavity being filled with a gas mixture, and the body part comprising a skin layer delimiting the cavity, the method comprising the steps of:
- placing an access element presenting a distal end so that the distal end is in communication with the cavity;
- measuring at least one gas mixture parameter related to the gas mixture within the cavity, with at least one sensor comprising a gas sensor arranged on the access element;
- compute the gas mixture parameter with a computational module connected to said at least one sensor, the computational module being configured to provide a gas information representative of the leakage in the anatomical conduit,
- communicate gas information to a user, with a user interface connected to the computational module.

According to invention, the detection device comprises a housing connected to the access element, the housing comprising:
- the computational module,
- the user interface, and
- a power supply configured to supply with energy the sensor, the computational module and the user interface,
and wherein the access element and the housing are configured to be held by hand by the user.

This method allows a quick and easy detection of leakage, when a surgery is already in progress.

### BRIEF DESCRIPTION OF THE DRAWINGS

[Fig.1] is a top view in perspective of an embodiment of the detection device configured to be mounted inside a cannula of a trocar, and featuring a user interface on an upper surface of a housing of the detection device.
[Fig.2] is a below view in perspective of the detection device of figure 1, showing an access element of the detection device, directly linked to the housing.
[Fig.3] is a cross section of the detection device of figure 1, showing components placed inside the housing, and a gas sensor placed inside a lumen of the access element.
[Fig.4] is a schematic view of the detection device of figure 1 and a trocar, before assembling.
[Fig.5] is a schematic view of the assembled detection device of figure 1 and trocar, illustrating the coupling of an adjustment member within the cannula for holding the detection device with the trocar, a sealing valve of the trocar pressed against the access element thus preventing leakage from the body cavity, and the sealing of an insufflating access hole of the trocar, thanks to a sealing member of the detection device.
[Fig.6] is a schematic view of the use of the detection device of figure 1 during a surgery, in which a hollow organ has been surgically treated, and is insufflated in order to check on the presence of a leakage on a suture made during the surgery, with the help of the detection device mounted in a trocar reaching a body cavity surrounding the hollow organ.
[Fig.7] is another schematic view of the use of the detection device during a surgery.

### DETAILED DESCRIPTION

The disclosure essentially concerns a handheld leakage detection device (1), for use in a surgery.

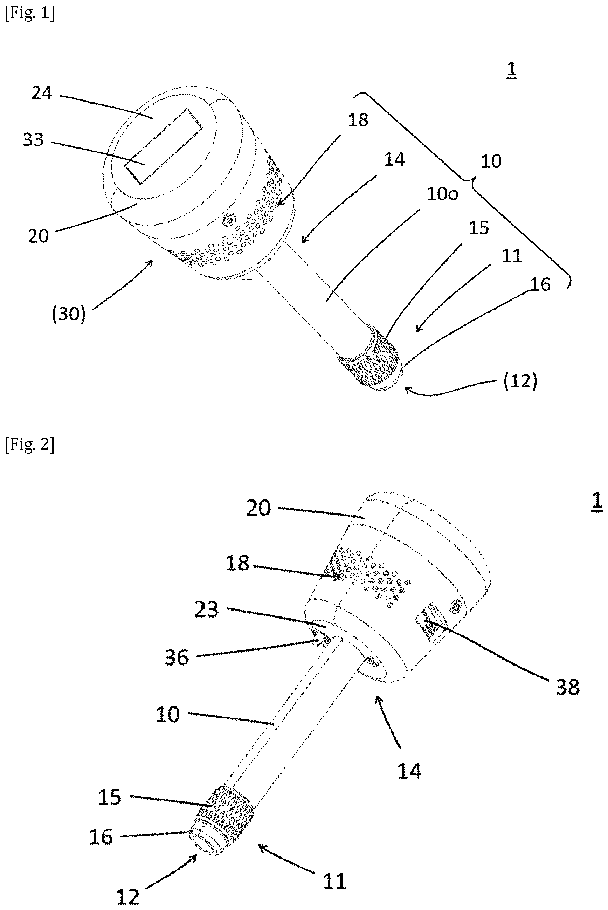

Figures 1 and 2 illustrate a preferred embodiment of the detection device (1), intended to cooperate with a trocar (100). It mainly comprises:
- an access element (10) intended to be in connection with the body cavity (3) wherein an organ (2), comprising an anatomical conduit, is under surgery,
- a housing (20) connected to the access element (10),
- a detection module (30).

The detection device (1) features an access element (10) extending along an axis, and comprising a proximal end (14) on the side of the housing (20), and a distal end (11) on a free side of the access element (10). The distal end (11) is configured to be in connection with the body cavity (3).

The access element (10) is preferably in the shape of a hollow cylinder revolving around the axis, traversed by a lumen (12), and extending from the distal end (11) in the direction to the proximal end (14), thus to the housing (20).

A gas sensor (31) is disposed within the lumen (12), thus allowing a gas mixture (GM) contained by the body cavity (3) to be in contact with the gas sensor (31). Said gas sensor (31) can then detect in the gas mixture (GM) the presence or an abnormal quantity of a test gas (TG) insufflated in the hollow organ (2), and which can have leaked from the insufflated hollow organ (2). The gas sensor (31) can also detect the pressure inside the body cavity (3).

In order to ensure the holding of the detection device (1), the access element (10) comprises an adjustment member (15), intended to be radially tight against the internal surface (111) of the cannula (110) of the trocar (100). To that end, the adjustment member (15) can be made of an elastomeric material. The adjustment member (15) can feature an external diameter slightly superior than an inner diameter of the internal surface (111).

The adjustment member (15) can be in the shape of a bush, placed in a circumferential recess of an outer surface (10o) of the access member (10). The adjustment member (15) can be of any other suitable shape, e.g., protrusions from the outer surface (10o) and so on.

The adjustment member (15) can further have a sealing function against the internal surface (111), preventing a leakage of the gas mixture (GM) through a radial space between the outer surface (10o) of the access element (10), and the internal surface (111) of the cannula (110).

In this case, an elastomeric material is preferred for the adjustment member (15). A bush shape is also preferred.

The proximal end (14) of the access element (10) is preferably mounted directly on the housing (20) which contains a computational module (32) of the detection device (1). This rigid and compact assembly allows the detection device (1) to be handled by a single hand.

An alternative not shown resides in a tubing connection through the access element (10) and the housing (20), with a flexible pipe. In order the detection device (1) remains handheld, the length of the pipe should not excess a few centimeters, e.g., 10 cm or 20 cm.

The housing (20) contains the computational module (32), for receiving the gas parameter acquired by the gas sensor (31), and process it. A user interface (33) is placed on the top (24) of the housing (20) to display the result of the analysis conducted by the computational module (32).

The user interface (33) can be a screen displaying an information. Means for the surgeon to prompt information for the computational module (32) may not be needed.

The user interface (33) can be a color-changing LED, the color of the LED indicating the potentiality of the presence of a leakage (L), according to a color-based code.

The user interface (33) can be a sound emitting module, configured to emit a sound alarm if the probability of a leakage (L) is above a predefined threshold.

The user interface (33) can be a wireless connection, as a Bluetooth connection, configured to display the information on an electronic device paired with the detection device (1), as a smartphone.

Preferably the user interface (33) is a screen, e.g., displaying the concentration of a specific gas among the gas mixture (GM). It can be the concentration of the test gas (TG), which will raise in case of a leakage (L). It can be the concentration of carbon dioxide, used to insufflate the body cavity (3). If a leakage (L) occurs, the test gas (TG) will replace some carbon dioxide among the gas mixture (GM). Thus, the concentration of carbon dioxide will decrease in case of a leakage (L).

The user interface (33) is preferably placed on a top side (24) of the housing (20), opposite to the access element (10), in order to be clearly visible from the surgeon point of view when the detection device (1) is mounted into the trocar (100).

In reference to figure 2, a switch (36) is placed on an under-face (23) of the housing (20). This allows the switch (36) to be activated by a top surface (102) of the trocar (100) head when the detection device (1) is mounted into the trocar (100). This switch (36) is used to turn the detection device (1) on and off: the detection device (1) according to this feature is by consequence a "plug and play" detection device (1), which automatically turns on when it is mounted into the trocar (100), and automatically turns off when the detection device (1) is dismounted from the trocar (100).

A such plug and play detection device (1) allows a very quick and easy installation and deinstallation during the surgery. It is thus easy to obtain information as to the presence of a leakage (L), on a regular basis, during the surgery.

This feature also helps to save the energy of the power supply (34) included in the housing (20).

The switch (36) can be placed elsewhere on the detection device (1), e.g., on the outer surface of the access element (10). In that case, the switch (36) can be activated by the mating inner surface (111) of the cannula (110) of the trocar (100).

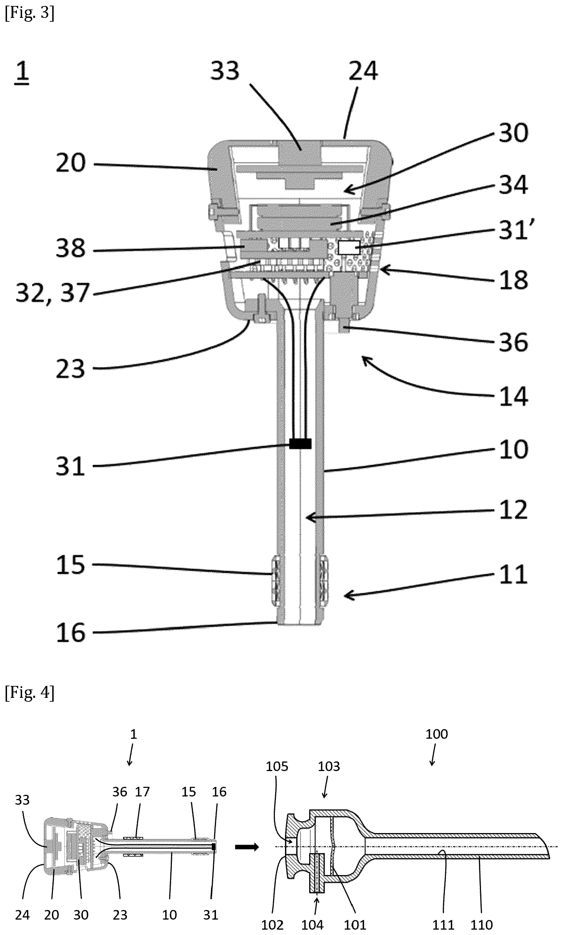

Referring to figure 3, the housing (20) includes:
- the computational module (32), which receives the sensing parameters and compute them to display a leakage information (LI),
- the user interface (33),
- and the power supply (34), for powering the computational module (32), the sensors (31, 31'), and so on.

Preferably, the housing (20) further includes a data storage device (37), and a data transfer interface (38). The latter is preferably a wire-type connector, in order to avoid the need to pair the detection device (1) with a computer, intended to receive the stored data after the surgery.

The housing (20) may further comprise a supplementary sensor (31'), intended to measure a supplementary gas parameter, as the pressure of the test gas (TG) insufflated in the hollow organ (2).

In that case, the detection device (1) features a fluidic connection with the insufflator (200), in order to link the supplementary sensor (31') with the insufflated test gas (TG). This connection can be easily made by adding a T shaped connector to the tube linking the insufflator (200) to the body, and directing a supplementary tube, connected to the T-shaped connector, to a connector of the housing (20).

Figure 4 illustrates the detection device (1) and the trocar (100), before their assembly.

The trocar (100) comprises a cannula (110) configured to penetrate the body cavity (3) through the skin (5), and a trocar (100) head which features:
- an access hole (105) designed to allow surgical instruments to access the body cavity (3) through the cannula (110),
- a sealing valve (101) preventing the leakage (L) of the gas mixture (GM) insufflated in the body cavity (3) to provide the needed space during the surgery,
- eventually, a connection hole (104) which can be used to insufflate the gas mixture (GM) into the body cavity (3). The presence of the connection hole (104) relies on the type of the trocar (100).

The access element (10) preferably comprises a passing member (16) configured to spread and pass the sealing valve (101) without damaging it. The passing member (16) can be a rounded shape, or a chamfer located at the distal end (11).

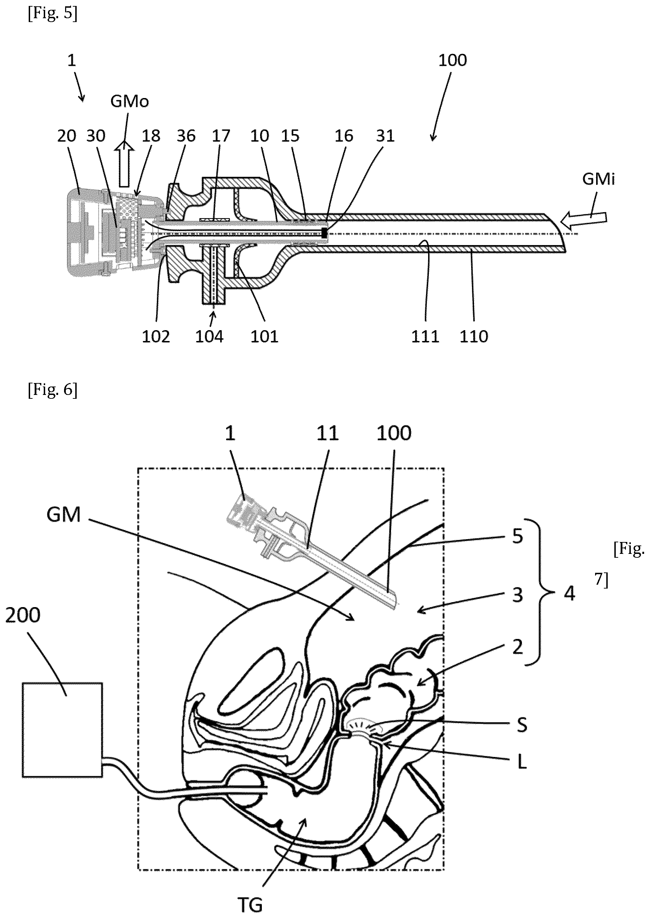

The figure 5 illustrates the detection device (1) mounted into the trocar (100).

The access element (10) has passed through the sealing valve (101) and has reached the cannula (110). The adjustment member (15) is radially pressing against the inner surface (111) of the cannula (110), thus preventing an unwanted dismounting of the detection device (1) and trocar (100).

Preferably, the sealing valve (101) is radially tight with the access element (10), in order to prevent unwanted leakage (L) of the gas mixture (GM). Moreover, this tightness contributes to the correct holding of the detection device (1).

A sealing member (17) is mating with the connection hole (104) and seals it, also to prevent the leakage (L) of the gas mixture (GM).

An upper surface (102) of the trocar head (103) has pushed the switch (36), automatically turning on the detection device (1), therefore starting the leakage analysis.

The housing (20) comprises a gas outlet (18) to generate a flow of the gas mixture (GM) from the body cavity (3) to the distal end (11), through the lumen (12), and then to the gas outlet (18).

The gas mixture (GM) flows from a gas inlet (GMi) at the distal end (11) to the gas outlet (18) where it exits the detection device (GMo). The gas sensor (31) being placed inside the lumen (12), it is surrounded by the gas mixture (GM) from the body cavity (3).

This flow is generated by the slight overpressure residing inside the body cavity (3) during this kind of surgery.

This flow further helps the potentially leaking test gas (TG) to leave the surroundings of the leakage (L), and to ensure the leaking test gas (TG) to reaches the gas sensor (31).

The gas outlet (18) can be adapted in size and geometry in order to adapt the flowrate of the gas mixture (GM) leaving the body cavity (3).

Figure 6 illustrates a leakage system being used during a surgery. At certain point during the surgery, the surgeon may want to verify if there is a leakage (L) on the organ (2) he is working on. He can remove the surgery instruments form the trocar (100), place instead the detection device (1) for the duration of the detection.

An insufflator (200) fills the hollow organ (2) with a test gas (TG), in order to verify if the suture (S) made by the surgeon presents a leakage (L).

If so, some test gas (TG) will reach the body cavity (3) and be present in the gas mixture (GM) herein. The distal end (11) of the detection device (1) being placed inside the cannula (110) of the trocar (100), the sensor (not shown on fig. 6) can acquire the needed test gas parameter (TGP).

According to the results, he can take appropriate measures, i.e., either carry on the surgery if no leakage (L) is occurring, or treat the organ (2) if a leakage (L) has been detected.

The surgeon can remove the detection device (1) and place back the surgery instruments instead. Using the same trocar (100) for these two purposes allows to avoid a further incision in the body to place the detection device (1). This method is preferred when a punctual surveillance is sufficient.

In alternative, when a continuous surveillance is required, the detection device (1) can be mounted on a dedicated trocar (100). The surveillance is more thorough, but the drawback is the need to make a supplementary incision in the body.

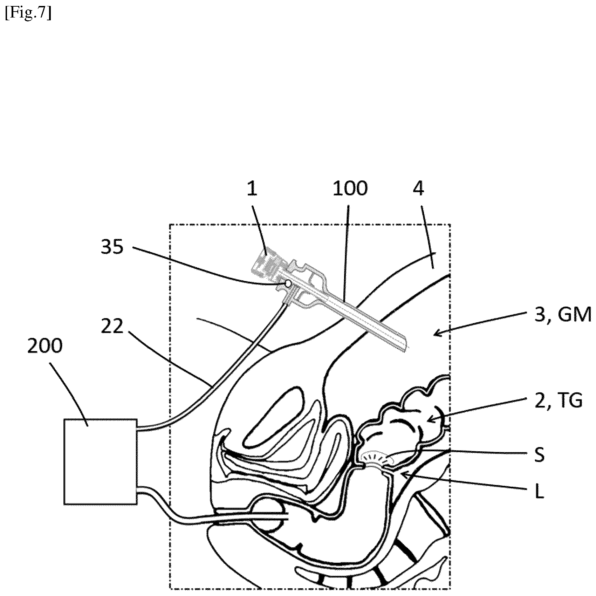

Figure 7 illustrates an embodiment wherein the housing (20) comprises a supplementary sensor (31') configured to measure a supplementary parameter, as the nature or the pressure of the insufflated test gas (TG) delivered to the detection device (1) through an insufflator tube (22).

In the represented embodiment of figures 6 and 7, although not limited thereto, the detection device (1) is implemented during a surgery, in particular a laparoscopy. The detection device (1) can however find other implementations in other surgeries or any other suitable purpose such as a follow-up further to a surgery.

In that case, the computational module (32) can proceed to further methods for leakage (L) detection, for example base on differential pressure as described in document EP3838119, in particular pages 5 to 6.

The supplementary sensor (31') can also be a temperature sensor, or any sensor needed.

Another embodiment allowing the use of a supplementary parameter reside in a wireless connection between the detection device (1) and the insufflator (200). In that case, the tube illustrated on figure 7 is no longer necessary, which is more ergonomic during the surgery. Nevertheless, it requires pairing the detection device (1) with the insufflator (200), so as the computational module (32) can communicate with the insufflator (200), and receive an external parameter from an external sensor of the insufflator (200).

The illustrated access element (10) is hollow and the gas sensor (31) is placed inside the lumen (12). The access element can also be a solid shaft. In that case, the gas sensor (31) can also be put on an outer surface of the access element (10).

In either way, once the detection device (1) is mounted on the trocar (100), the gas sensor (31) is in contact with the gas mixture (GM) and operable for the gas detection.

For easing the understanding of the invention, only the cooperation with a trocar (100) has been illustrated, but it is to be understood that the invention relies on the handheld and the autonomous features of the detection device (1), which can be of any form or any conception.

The detection device (1) can be in the shape of an all-in-one leakage detecting trocar, or a Veress needle, wherein the access element (10) is configured to penetrate the body cavity (3) by cutting the skin (5).

In addition, the technical features of the various embodiments and variants mentioned above can be combined with one another, either in whole or in part. In this way, the detecting device, the system and the method can be adapted in terms of cost, functionality and performance.

## Claims

1. Detection device (1) for use in providing a gas information (GI) from a gas mixture (GM) contained within a cavity (3) of a body part of a living being (4), and the body part comprising a skin layer (5) delimiting the cavity (3), the detection device (1) comprising:
- an access element (10) presenting a distal end (11) and configured to be in communication with the cavity (3),
- a housing (20) connected to the access element (10),
- a detection module (30),
wherein the access element (10) and the housing (20) are configured to be held by hand of a user, and the detection module (30) comprises:
- at least one sensor (31) comprising a gas sensor arranged on the access element (10) and configured to measure at least one gas mixture parameter (GMP) related to the gas mixture (GM) within the cavity (3),
- a computational module (32) arranged in the housing (20) and connected to said at least one sensor (31), the computational module (32) being configured to compute the gas mixture parameter (GMP) to provide the gas information (GI),
- a user interface (33) arranged in the housing (20) and connected to the computational module (32), the user interface (33) configured to communicate gas information (GI),
- a power supply (34) arranged in the housing (20) and configured to supply the detection module (30) with energy.

2. Detection device (1) according to claim **1,** wherein the computational module (32) is configured to communicate and receive an external parameter from an external sensor, and the computational module (32) is configured to compute the external parameter along with the gas mixture parameter (GMP).

3. Detection device (1) according to claims **1 to 2,** wherein it comprises a supplementary sensor (31') configured to measure at least one supplementary parameter, and the computational module (32) is configured to compute the supplementary parameter along with the gas mixture parameter (GMP).

4. Detection device (1) according to claims **1 to 3,** wherein the access element (10) comprises a gas outlet (17) at a proximal end (14) opposite the distal end (11).

5. Detection device (1) according to claims **1 to 4,** wherein it comprises a gas connection device (22) configured to:
- communicate with a test gas insufflator (200), and
- receive a test gas parameter (TGP),
and the computational module (32) is configured to compute the test gas parameter along with the gas mixture parameter (GMP).

6. Detection device (1) according to claims **1 to 5,** wherein the access element (10) presents a proximal end (14) opposite the distal end (11), the housing (20) being attached to the proximal end (14) of the access element (10).

7. Detection device (1) according to claims **1 to 6,** wherein the housing (20) comprises a data storage (37), configured to store gas parameter data from the sensor (31), or configured to store gas information (GI).

8. Detection device (1) according to claims **1 to 7,** wherein the gas information (GI) is representative of a leakage (L) in an anatomical conduit (2) within the cavity (3).

9. Detection device (1) according to claims **1 to 8** for use with a trocar (100) having a cannula (110) defining a passage to a free end and intended to be inserted in the body part (4) through the skin layer (5), wherein the access element (10) is configured to penetrate the cannula (110) with the gas sensor (31) in fluid communication with the passage, so that the gas sensor (31) is in contact with the gas mixture (GM) when the cannula (110) is arranged within the cavity (3).

10. Detection device (1) according to claim **9,** wherein an adjustment member (15) disposed on an outer surface of the access element (10) is configured to be radially tight against an internal surface (111) of the cannula (110).

11. Detection device (1) according to claims **9 to 10,** wherein the access element (10) is configured to be radially tight against a sealing valve (101).

12. Detection device (1) according to claims **9 to 11,** wherein a switch (36) controlling an activation and a deactivation of the detection module (30) is arranged on an external surface of the detection module (30), and is configured to be actuated by the trocar (100) when the detection module (30) is mounted on the trocar (100).

13. Detection device (1) according to claims **9 to 12,** wherein a sealing member (17) on an outer surface of the access element (10) is configured to obturate a connection hole (104) of the trocar (100) configured to insufflate the gas mixture (GM) into the body cavity (3), when the access element (10) penetrates the cannula (110).

14. System comprising:
- a trocar (100), comprising a cannula (110) which is intended to be inserted, through a skin layer (5) delimiting a cavity (3) of a body part of a living being (4), so that the cannula (110) is arranged within the cavity (3) which is filled by a gas mixture (GM); and
- a handheld detection device (1) according to claims **9 to 13,** and configured to be inserted in the cannula (110) so that the distal end (11) is in communication with the cavity (3), and so that the sensor (31) measures at least one gas mixture parameter (GMP) related to the gas mixture (GM).

15. System comprising:
- an insufflator (200) intended to inject a test gas (TG) into an anatomical conduit (2) within the cavity (3)
- a handheld detection device (1) according to **claim 5,**
so that the sensor (31) measures at least one gas mixture parameter (GMP) related to the test gas (TG).
